# EUROPEAN PATENT APPLICATION

(11) **EP 1 647 271 A1**
(43) Date of publication of application: **19.04.2006**
(21) Application number: 04292447.2
(22) Date of filing: 14.10.2004
(51) Int. Cl.: A61K 31/138, A61K 9/06, A61K 47/38, A61K 47/10, A61K 47/14, A61P 35/00

(54) **4-Hydroxy tamoxifen gel formulations**

(71) Applicant: LABORATOIRES BESINS INTERNATIONAL, 75003 Paris (FR)
(72) Inventor: Masini-Eteve, Valérie, 91370 Verrieres Le Buisson (FR)
(74) Representative: Nargolwalla, Cyra

(57) **Abstract**

The present invention relates to (4-hydroxy tamoxifen)-containing pharmaceutical compositions and gels, and to methods using the same.

## Description

The present invention relates to (4-hydroxy tamoxifen)-containing pharmaceutical compositions and gels, and to methods using the same.

The compound 4-hydroxy tamoxifen (herein after referred to as 4-OHT) or 1-[4-(2-N-dimethylaminoethoxy)phenyl]-1-(4-hydroxyphenyl)-2-phenylbut-1-ene, constitutes an active metabolite of the well characterized anti-estrogen compound, tamoxifen. Due to the presence of a double bond between two carbon atoms, 4-hydroxy tamoxifen exists in two stereoisomeric forms. According to the medical and biochemical literature, isomeric forms of 4-hydroxy tamoxifen are commonly designated as cis and trans isomers. From a purely chemical perspective, however, this designation is not strictly accurate because each double bonded carbon atom does not contain an identical chemical group. Therefore, it is more appropriate to refer to the isomers as (E) (the so-called cis form) and (Z) (the so-called trans form) configurations.

US 4 919 937 to Mauvais-Jarvis et al discloses 4-OHT formulations. However, these formulations do not contain any penetration enhancer, and are specifically designed for the co-administration of 4-OHT together with progesterone.

There is a need for improved 4-OHT containing formulations, which are easy to use for the patient, with easy compliance, allow reproducible delivery of 4-OHT, with improved safety profiles and decreased systemic effects. There is also a need for 4-OHT topical formulations, which allow better penetration of the active ingredient across the skin.

The present invention provides 4-OHT-containing pharmaceutical compositions and gels, and methods for using the same. More particularly, the present invention provides hydroalcoholic pharmaceutical compositions suitable for topical and transdermal application, wherein said compositions contain 0.105 - 0.950 % of 4-hydroxy tamoxifen by weight based on the total weight of the composition.

In addition to achieving a very effective 4-OHT delivery, alone or in combination with another medicament, the compositions of the invention exhibit improved safety profiles, allow easy patient compliance, and show decreased systemic exposure.

The present invention thus relates to a pharmaceutical composition comprising 4-OHT, at least one C2-C6 alcohol, at least one gelling agent, at least one penetration enhancer, and an aqueous vehicle.

Said pharmaceutical composition can be made in various forms, e.g. a gel, a solution, a cream, a lotion, a spray, an ointment, an aerosol. Preferably, said pharmaceutical composition is a gel, and is suitable for topical administration.

The term "4-OHT" (4-hydroxy tamoxifen) as used therein refers to 1-[4-(2-N-dimethylaminoethoxy)phenyl]-1-(4-hydroxyphenyl)-2-phenylbut-1-ene. 4-OHT encompasses both the (Z) isomer and the (E) isomer, as well as mixtures thereof, including racemic and non racemic mixtures. The (Z) isomer is preferred because it is more active than the (E) isomer.

C2-C6 alcohols are known in the art. Such alcohols encompass ethanol, propanol, isopropanol (propan-2-ol), n-propanol (propan-1-ol), butanol, butan-1-ol, butan-2-ol, ter-butanol, pentanols, hexanols. Ethanol is preferred, since it contributes efficiently towards the transdermal passage of 4-OHT by evaporating quickly on contact with the skin.

Gelling agents are known in the art. The term 'gelling agent' generally refers to a compound, possibly of polymeric nature, having the capacity to gel when contacted with a specific solvent, e.g. water. Gelling agents make it possible to increase the viscosity of the pharmaceutical compositions according to the invention, but may also act as solubilizing agents. Examples of gelling agents include anionic polymers such as acrylic acid based polymers (including polyacrylic acid polymers, e.g. CARBOPOL® by B.F. Goodrich Specialty Polymers and Chemicals Division of Cleveland, Ohio)., cellulose derivatives, poloxamers and poloxamines, more precisely, Carbomers or acrylic acid-based polymers, e.g. Carbopol® 980 or 940, 981 or 941, 1382 or 1382, 5984, 2984, 934 or 934P (Carbopol® are usually polymers of acrylic acid crosslinked with allyl sucrose or allylpentaerythritol), Pemulen TR1 ® or TR2 ®, Ultrez, Synthalen CR, etc.); cellulose derivatives such as carboxymethylcelluloses, hydroxypropylcelluloses, hydroxyethylcelluloses, ethylcelluloses, hydroxymethylcelluloses, hydroxypropylmethylcelluloses, and the like, and mixtures thereof; poloxamers or polyethylene-polypropylene copolymers such as Lutrol ® grade 68 or 127, poloxamines and other gelling agents such as chitosan, dextran, pectins, and natural gums. All of these gelling agents, alone or in combination, may be used in the pharmaceutical composition according to the invention. Cellulosics, including carboxymethylcelluloses, hydroxypropylcelluloses, hydroxyethylcelluloses, ethylcelluloses, hydroxymethylcelluloses, hydroxypropylmethylcelluloses, and mixtures thereof, are particularly preferred in the context of the present invention.

Penetration enhancers are also known in the art. A 'penetration enhancer' is generally an agent known to accelerate the delivery of the drug or active principle through the skin. These agents also have been referred to as penetration accelerants, adjuvants, and absorption promoters. This class of agents includes those with diverse mechanisms of action including those which have the function of improving the solubility and diffusibility of the drug, and those which improve transdermal absorption by changing the ability of the stratum corneum to retain moisture, softening the skin, improving the skin's permeability, acting as penetration assistants or hair-follicle openers or changing temporarily the state of the skin such as the boundary layer. The penetration enhancer within the context of the present invention can be a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms. Non-limiting examples of penetration enhancers include C8-C22 fatty acids such as isostearic acid, octanoic acid, and oleic acid; C8-C22 fatty alcohols such as oleyl alcohol and lauryl alcohol; lower alkyl esters of C8-C22 fatty acids such as ethyl oleate, isopropyl myristate, butyl stearate, and methyl laurate; di(lower)alkyl esters of C6-C8 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; glycol ethers including diethylene glycol monoethyl ether; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; pyrrolidones and N-alkylpyrrolidone; terpenes; hydroxyacids; urea; essential oils; and mixtures thereof. Preferred examples include myristate isopropyl. All of these penetration enhancers can be used either alone or in combination, e.g. combination of two or three different penetration enhancers.

Aqueous vehicles are known in the art. According to another aspect of the invention, said aqueous vehicle comprises, besides water, ingredients useful in adjusting pH, for instance at least one buffering agent. Buffering agents, especially pharmacologically acceptable buffering agents, are known in the art. In one aspect, said aqueous vehicle comprises at least one buffer, preferably selected from the group consisting of citrate buffers, e.g. sodium citrate and/or potassium citrate; tris buffers, e.g. tris maleate; phosphate buffers, including Sorensen-type buffers, dibasic or monobasic phosphate, e.g. sodium dibasic or monobasic phosphate.

In another aspect, the pharmaceutical composition of the invention further comprises a base. Advantageously, said base is preferably pharmaceutically acceptable, and is preferably selected from the group consisting of triethanolamine, sodium hydroxide, ammonium hydroxide, potassium hydroxide, arginine, aminomethylpropanol or tromethamine, and mixtures thereof. Where the pH of said pharmaceutical composition is not optimized for transdermal administration, e.g. where said gelling agent comprises at least one acrylic acid-based polymer, said base contributes to the neutralization of said pharmaceutical composition, for topical application onto human skin. Furthermore, said base (neutralizer) allows optimum swelling of the polymer chains during the neutralization of the charges and the formation of polymer salts. Especially when said gelling agent comprises an acrylic acid-based polymer, said base preferably comprises triethanolamine. It also allows an optimum viscosity to be achieved in the pharmaceutical composition according to the invention. The skilled person would know how to choose a suitable amount of said base in the composition, especially with respect to the nature of said gelling agent present therein, and the alcohol content of the composition, in order to reach the desired final pH in the composition. For example, with carbomers and/or if there is a high alcohol content, one can use tromethamine and/or NaOH as a base, in amounts chosen as to reach the desired final pH in the composition. The base/gelling agent ratio preferably is between 10:1 and 0.1:1, more preferably between 7:1 and 0.5:1, and still more preferably between 4:1 and 1:1.

Unless otherwise stated, percentages (%) refer to amounts by weight based upon total weight of the composition. Amounts of alcohol(s) herein refer to absolute alcohol content, e.g. absolute ethanol content.

According to one aspect, said pharmaceutical composition comprises 0.105-0.950%, preferably 0.105-0.195%, more preferably 0.105-0.185%, more preferably 0.105-0.175%, more preferably 0.105-0.165%, more preferably 0.105-0.155%, more preferably 0.105-0.145%, more preferably 0.105-0.135%, more preferably 0.105-0.125%, more preferably 0.107-0.119%, more preferably 0.111-0.118%, more preferably 0.112-0.117%, still more preferably 0.113-0.116%, most preferably 0.114-0.115% of 4-OHT.

According to another aspect, said pharmaceutical composition comprises 0.205-0.950%, preferably 0.210-0.900%, more preferably 0.215-0.800%, even more preferably 0.220-0.750%, even more preferably 0.220-0.700%, even more preferably 0.220-0.600%, even more preferably 0.220-0.500%, still more preferably 0.220-0.400%, most preferably 0.220-0.350% of 4-OHT.

According to another aspect of the invention, said pharmaceutical composition comprises 40-80%, preferably 45-75%, more preferably 50-75%, still more preferably 55-75%, most preferably 60-75% of at least one alcohol.

According to another aspect of the invention, said C2-C6 alcohol is selected from the group consisting of ethanol, propan-1-ol and propan-2-ol, and mixtures thereof. Preferably said C2-C6 alcohol comprises ethanol.

According to another aspect of the invention, said pharmaceutical composition comprises 0.1-5.0 %, preferably 0.15-4.5 %, more preferably 0.2-4.0 %, even more preferably 0.25-3.5 %, even more preferably 0.3-3.0 %, even more preferably 0.4-2.5 %, still more preferably 0.5-2.0 %, most preferably about 0.5-1.5 % of at least one gelling agent.

According to another aspect of the invention, said gelling agent comprises at least one selected from the group of cellulosics, including cellulose esters and derivatives (cellulose derivatives such as carboxymethylcelluloses, hydroxypropylcelluloses, hydroxyethylcelluloses, ethylcelluloses, hydroxymethylcelluloses, hydroxypropylmethylcelluloses, and the like); and mixtures thereof. Preferably, said gelling agent is selected from carboxymethylcelluloses, hydroxypropylcelluloses, hydroxyethylcelluloses, ethylcelluloses, hydroxymethylcelluloses, hydroxypropylmethylcelluloses, and mixtures thereof. Most preferably, said gelling agent comprises hydroxypropylcellulose, e.g. Klucel.

According to another aspect of the invention, said pharmaceutical composition comprises 0.1-5.0 %, preferably 0.15-4.5 %, more preferably 0.2-4.0 %, even more preferably 0.25-3.5 %, even more preferably 0.3-3.0 %, even more preferably 0.4-2.5 %, still more preferably 0.5-2.0 %, most preferably about 0.5-1.5 % of at least one penetration enhancer.

According to another aspect of the invention, said pharmaceutical composition comprises 0.4-2.0 %, preferably 0.5-1.9 %, more preferably 0.5-1.8 %, even more preferably 0.5-1.7 %, even more preferably 0.5-1.6 %, even more preferably 0.5-1.5 %, even more preferably 0.6-1.4 %, even more preferably 0.7-1.3 %, still more preferably 0.8-1.2 %, most preferably about 0.9-1.1 %, or 1.0 % of myristate isopropyl.

The compositions according to the invention may include several penetration enhancers.

According to another aspect of the invention, said pharmaceutical composition comprises 20-50 %, preferably 20-40 % of an aqueous vehicle.

According to another aspect of the invention, said aqueous vehicle preferably comprises at least one buffer, especially a phosphate buffer.

In another aspect of the invention, the pH of the pharmaceutical composition is preferably 7-11, more preferably 7.5-10.5, even more preferably 8.0-10.0, still more preferably 8.5-10.0, most preferably about 8.5-9.5.

In one embodiment, the present invention provides a pharmaceutical composition comprising:
- 0.205-0.950 % (respectively 0.105-0.950%) of 4-OHT,
- 40-80 % of a least one C2-C6 alcohol, preferably ethanol,
- 0.1-5.0 % of at least one gelling agent, preferably hydroxypropylcellulose,
- 0.1-5.0 % of at least one penetration enhancer,
- 20-50 % of an aqueous vehicle.

In another embodiment, the present invention provides a pharmaceutical composition comprising:
- 0.210-0.900 % (respectively 0.105-0.185%) of 4-OHT,
- 40-75 % of a least one C2-C6 alcohol, preferably ethanol,
- 0.15-4.5 % of at least one gelling agent, preferably hydroxypropylcellulose,
- 0.15-4.5 % of at least one penetration enhancer,
- 20-40 % of an aqueous vehicle.

In another embodiment, the present invention provides a pharmaceutical composition comprising:
- 0.215-0.800 % (respectively 0.105-0.165%) of 4-OHT,
- 45-75 % of a least one C2-C6 alcohol, preferably ethanol,
- 0.2-4.0 % of at least one gelling agent, preferably hydroxypropylcellulose,
- 0.2-4.0 % of at least one penetration enhancer,
- 20-40 % of an aqueous vehicle.

In another embodiment, the present invention provides a pharmaceutical composition comprising:
- 0.220-0.750 % (respectively 0.105-0.145%) of 4-OHT,
- 45-75 % of a least one C2-C6 alcohol, preferably ethanol,
- 0.25-3.5 % of at least one gelling agent, preferably hydroxypropylcellulose,
- 0.25-3.5 % of at least one penetration enhancer,
- 20-40 % of an aqueous vehicle.

In another embodiment, the present invention provides a pharmaceutical composition comprising:
- 0.220-0.700 % (respectively 0.105-0.125%) of 4-OHT,
- 50-75 % of a least one C2-C6 alcohol, preferably ethanol,
- 0.3-3.0% of at least one gelling agent, preferably hydroxypropylcellulose,
- 0.3-3.0% of at least one penetration enhancer,
- 20-40 % of an aqueous vehicle.

In another embodiment, the present invention provides a pharmaceutical composition comprising:
- 0.220-0.600 % (respectively 0.111-0.118%) of 4-OHT,
- 60-75 % of a least one C2-C6 alcohol, preferably ethanol,
- 0.4-2.5 % of at least one gelling agent, preferably hydroxypropylcellulose,
- 0.4-2.5 % of at least one penetration enhancer,
- 20-40 % of an aqueous vehicle.

In another embodiment, the present invention provides a pharmaceutical composition comprising:
- 0.220-0.500 % (respectively 0.112-0.117%) of 4-OHT,
- 60-75 % of a least one C2-C6 alcohol, preferably ethanol,
- 0.5-2.0 % of at least one gelling agent, preferably hydroxypropylcellulose,
- 0.5-2.0 % of at least one penetration enhancer,
- 20-40 % of an aqueous vehicle,

In another embodiment, the present invention provides a pharmaceutical composition comprising:
- 0.220-0.400 % (respectively 0.113-0.116%) of 4-OHT,
- 60-75 % of a least one C2-C6 alcohol, preferably ethanol,
- 0.5-1.5 % of at least one gelling agent, preferably hydroxypropylcellulose,
- 0.5-1.5 % of at least one penetration enhancer,
- 20-40 % of an aqueous vehicle.

In another embodiment, the present invention provides a pharmaceutical composition comprising:
- 0.220-0.350 % (respectively 0.114-0.115%) of 4-OHT,
- 60-75 % of a least one C2-C6 alcohol, preferably ethanol,
- 0.5-1.5 % of at least one gelling agent, preferably hydroxypropylcellulose,
- 0.5-1.5 % of at least one penetration enhancer,
- 20-40 % of an aqueous vehicle.

In another embodiment, the present invention provides a pharmaceutical composition comprising:
- 0.205-0.950 % (respectively 0.105-0.950%) of 4-OHT,
- 40-80 % of a least one C2-C6 alcohol, preferably ethanol,
- 0.1-5.0 % of at least one gelling agent, preferably hydroxypropylcellulose,
- 0.4-2.0 % of myristate isopropyl,
- 20-50 % of an aqueous vehicle.

In another embodiment, the present invention provides a pharmaceutical composition comprising:
- 0.210-0.900 % (respectively 0.105-0.185%) of 4-OHT,
- 40-75 % of a least one C2-C6 alcohol, preferably ethanol,
- 0.15-4.5 % of at least one gelling agent, preferably hydroxypropylcellulose,
- 0.5-1.8 % of myristate isopropyl,
- 20-40 % of an aqueous vehicle.

In another embodiment, the present invention provides a pharmaceutical composition comprising:
- 0.215-0.800 % (respectively 0.105-0.165%) of 4-OHT,
- 45-75 % of a least one C2-C6 alcohol, preferably ethanol,
- 0.2-4.0 % of at least one gelling agent, preferably hydroxypropylcellulose,
- 0.6-1.4 % of myristate isopropyl,
- 20-40 % of an aqueous vehicle.

In another embodiment, the present invention provides a pharmaceutical composition comprising:
- 0.220-0.750 % (respectively 0.105-0.145%) of 4-OHT,
- 45-75 % of a least one C2-C6 alcohol, preferably ethanol,
- 0.25-3.5 % of at least one gelling agent, preferably hydroxypropylcellulose,
- 0.5-1.5 % of myristate isopropyl,
- 20-40 % of an aqueous vehicle.

In another embodiment, the present invention provides a pharmaceutical composition comprising:
- 0.220-0.700 % (respectively 0.105-0.125%) of 4-OHT,
- 50-75 % of a least one C2-C6 alcohol, preferably ethanol,
- 0.3-3.0% of at least one gelling agent, preferably hydroxypropylcellulose,
- 0.6-1.4 % of myristate isopropyl,
- 20-40 % of an aqueous vehicle.

In another embodiment, the present invention provides a pharmaceutical composition comprising:
- 0.220-0.600 % (respectively 0.111-0.118%) of 4-OHT,
- 60-75 % of a least one C2-C6 alcohol, preferably ethanol,
- 0.4-2.5 % of at least one gelling agent, preferably hydroxypropylcellulose,
- 0.7-1.3 % of myristate isopropyl,
- 20-40 % of an aqueous vehicle.

In another embodiment, the present invention provides a pharmaceutical composition comprising:
- 0.220-0.500 % (respectively 0.112-0.117%) of 4-OHT,
- 60-75 % of a least one C2-C6 alcohol, preferably ethanol,
- 0.5-2.0 % of at least one gelling agent, preferably hydroxypropylcellulose,
- 0.8-1.2 % of myristate isopropyl,
- 20-40 % of an aqueous vehicle,

In another embodiment, the present invention provides a pharmaceutical composition comprising:
- 0.220-0.400 % (respectively 0.113-0.116%) of 4-OHT,
- 60-75 % of a least one C2-C6 alcohol, preferably ethanol,
- 0.5-1.5 % of at least one gelling agent, preferably hydroxypropylcellulose,
- 0.9-1.1 % of myristate isopropyl,
- 20-40 % of an aqueous vehicle.

In another embodiment, the present invention provides a pharmaceutical composition comprising:
- 0.220-0.350 % (respectively 0.114-0.115%) of 4-OHT,
- 60-75 % of ethanol,
- 0.5-1.5 % of hydroxypropylcellulose,
- 0.4-2.0 % of myristate isopropyl,
- 20-40 % of an aqueous vehicle.

Additionally, said pharmaceutical composition may comprise usual pharmaceutical additives, including salt(s), emollient(s), stabilizer(s), antimicrobial(s), fragrance(s), and/or propellant(s). Said pharmaceutical composition may also include at least one further active ingredient.

The invention also provides a gel useful for transdermal or transcutaneous delivery comprising said pharmaceutical composition according to the invention. Thus, the invention is also directed to a 4-OHT-containing hydro-alcoholic gel.

According to another embodiment, the present invention provides a dose packet, unit dose packet or multiple dose packet containing said pharmaceutical composition or said hydroalcoholic gel. Advantageously, such conditioning of said pharmaceutical composition renders application easier for a patient. Thus, these forms of packaging can reflect the schedule of application, e.g. daily or weekly administration.

According to another embodiment, there is provide a dispenser, e.g. with hand pump or valve, containing said pharmaceutical composition or said hydroalcoholic gel. Such dispensers allow for flexibility in the administered dosage, as a function of the amount of composition to be applied.

According to one embodiment, said packets or dispensers can be accompanied by a notice giving instructions for the use thereof.

The pharmaceutical compositions, gels, packets and containers of the invention are useful for treating various conditions and/or disorders.

The term 'treat' or 'treatment' as used herein refers to any treatment of a mammalian condition, disorder, or disease associated with a depressive disorder, and includes, but is not limited to, preventing the condition, disorder, or disease from occurring in a subject which may be predisposed to the condition, disorder, or disease, but has not yet been diagnosed as having the condition, disorder, or disease; inhibiting the condition, disorder, or disease, for example, arresting the development of the condition, disorder, or disease; relieving the condition, disorder, or disease, for example, causing regression of the condition, disorder, or disease; or relieving the condition caused by the disease or disorder, for example, stopping the symptoms of the disease or disorder.

The term 'prevent' or 'prevention', in relation to a condition, disorder, or disease, means no condition, disorder, or disease development if none had occurred, or no further condition, disorder, or disease development if there had already been development of the condition, disorder, or disease.
In particular, the pharmaceutical compositions, gels, packets and containers of the invention are useful for:
- treating and/or preventing conditions involving dense breast tissue. High density breast tissues are a predictor of breast cancer risk, and compromises mammographic sensitivity, which is a major issue for cancer detection and diagnostic. Said dense breast tissue can be diffuse or nodular;
- treating and/or preventing benign breast diseases. Benign breast disease generally refers to a constellation of common non-malignant aberrations in breast tissue. These aberrations include numerous lesions that have well-defined histological characteristics, and can be classified as proliferative or nonproliferative. Exemplary benign breast diseases treatable by the present methods include adenosis, cysts, duct ectasia, fibroadenoma, fibrosis, hyperplasia, metaplasia and other fibrocystic changes. Each of these diseases, often referred to as "changes" or "conditions" due to their prevalence, have well-defined histological and clinical characteristics. "Adenosis" refers to generalized glandular disease of the breast. It typically involves an enlargement of breast lobules, which contain more glands than usual. In "sclerosing adenosis," or "fibrosing adenosis," the enlarged lobules are distorted by scar-like fibrous tissue. "Cysts" are abnormal sacs filled with fluid or semi-solid material, and lined by breast epithelial cells, developing from lobular structures. They begin as excess fluid inside breast glands, but may grow to proportions that stretch surrounding breast tissue, causing pain. "Fibrocysts" are cystic lesions circumscribed by, or situated within, a conspicuous amount of fibrous connective tissue. "Duct ectasia" refers to a dilation of mammary ducts by lipid and cellular debris. Rupture of the ducts induces infiltration by granulocytes and plasma cells. "Fibroadenoma" refers to benign tumors that are derived from glandular epithelium and contain a conspicuous stroma of proliferating fibroblasts and connective tissue. "Fibrosis" simply refers to a prominence of fibrous tissue in the breast. "Hyperplasia" refers to an overgrowth of cells, where several layers of cells line the basal membrane, without tumor formation. Hyperplasia increases the bulk of mammary tissue. In "epithelial hyperplasia," the cells lining breast ducts and lobules are involved, giving rise to the terms "ductal hyperplasia" and "lobular hyperplasia." Based on a histological determination, hyperplasia may be characterized as "usual" or "atypical". "Metaplasia" refers to a phenomenon in which a differentiated tissue of one type transforms into a differentiated tissue of another type. Metaplasia often results from an environmental change, and enables cells better to withstand the change;
- preventing and/or treating scars, including excessive scarring, keloid scars and hypertrophic scars. "Excessive scar" or "excessive scarring" generally refers to overgrowths of dense fibrous tissue that result from abnormal wound healing. Excessive scars have grown larger than necessary for normal wound healing, and are characterized by overproduction of cells, collagen and/or proteoglycan. "Keloid scars" are excessive scars in which the dense fibrous tissue extends beyond the borders of the original wound or incision, and does not usually regress spontaneously. Determining whether a scar is a keloid can be difficult, since keloids often superficially resemble other hypertrophic scars. However, keloids have distinguishing histological features. One such feature is the collagen nodule, which contains a high density of fibroblasts and unidirectional collagen fibrils in a highly organized and distinct orientation. Additionally, keloids have a rich vasculature, a high mesenchymal cell density, and a thickened epidermal cell layer. "Hypertrophic scars" are excessive scars in which the dense fibrous tissue does not extend beyond the borders of the original wound or incision. They tend to be wider than necessary for normal wound healing to occur. Histologically, hypertrophic scars have more organized collagen fibers than keloids, and scant mucoid matrix. Hypertrophic lesions are characterized by randomly distributed tissue bundles consisting of uniaxially oriented extracellular matrix and cells;
- treating gynecomastia. Gynecomastia is a common clinical condition, often presenting secondarily to an underlying disorder, representing the benign and sometimes painful proliferation of breast tissue in young boys and adult males;
- preventing and/or treating breast cancer, especially non-invasive breast cancer;
- treating mastalgia. Mastalgia, also called "mastodynia" or breast pain constitutes the most common breast problem for which women consult general medical practitioners. Its severity varies, but mastalgia can be so prolonged and intense as to interfere with normal daily activities, and even to disable afflicted individuals. Mastalgia can be classified according to three general sources of pain: (1) cyclical mammary pain, (2) non-cyclical mammary pain, and (3) extramammary pain. Cyclical mastalgia results from physiological breast enlargement, caused by estrogen-dependent vascular changes, during the luteal phase of the menstrual cycle, and affects a majority of premenopausal women. Cyclical mastalgia also can recur in postmenopausal women on estrogen replacement therapy, with a dose-dependent effect. "Non-cyclical mastalgia", as its name suggests, refers to pain in the breast that is not related to the menstrual cycle. A number of conditions give rise to non-cyclical mastalgia, including sclerosing adenosis, Tietz's syndrome and, rarely, breast cancer. Finally, extramammary mastalgia includes breast pain that is projected to the breast from other sources, as occurs, for example, when a patient feels pain from muscles or ribs that underlie the breasts.

The present pharmaceutical compositions and gels can also be used in "combination therapy" with a further active agent.

In another aspect, the invention also relates to the use of the gel or the solution according to the invention for the preparation of a medicinal product for transdermal application for the treatment of at least one of the above-mentioned indications.

In another aspect, the present invention relates to a method of treatment.

In one embodiment, said method comprises the step of administering 4-OHT topically, to a subject in need thereof.

According to one aspect, the pharmaceutical composition or the gel of the present invention is applied on one breast, or both, or on the scar, depending upon the indication.

The 4-OHT dosage can be adjusted by the one skilled in the art. When applied onto breast skin, this can be 0.25-3.0 mg/day/breast, preferably 0.50-2.5 mg/day/breast, more preferably 0.75-2.0 mg/day/breast, even more preferably 1.0-1.5 mg/day/breast. When applied onto a scar, this can be 0.25-3.0 µg/cm², preferably 0.5-2.5 µg/cm², more preferably 1.0-2.0µg/cm².

The pharmaceutical compositions and methods of the invention are particularly useful for patients, especially women, to the extent that they allow an easy and reproducible compliance. The amount of 4-OHT absorbed within the context of the present no longer depends on the size of the patient's breast (or scars, respectively). Contrary to what happens with less concentrated formulations, the compositions and methods of the invention result in a good control of the 4-OHT absorbed dose, and thus allow easy compliance for the patient. This results in less variability. Moreover, the number of required applications is decreased (e.g. once a day), and the 4-OHT systemic exposure is herewith minimized, leading to decreased potential side effects.

In another aspect, the invention provides a process for preparing said pharmaceutical composition or gel according to the invention.

In one aspect, said process comprises the step of preparing a mixture containing at least one C2-C6 alcohol, and at least one penetration enhancer.

In another aspect, said process comprises the step of adding 4-OHT in the desired amount, and mixing.

In another aspect, said process comprises the step of adding at least one gelling agent, and mixing.

In another aspect, said process comprises the step of adding at least one aqueous vehicle, and mixing.

In another aspect, said process comprises the steps of:
- preparing a mixture containing at least one C2-C6 alcohol, and at least one penetration enhancer;
- adding 4-OHT in the desired amount, and mixing;
- adding at least one gelling agent, and mixing again; and
- adding at least one aqueous vehicle, and mixing again. The skilled person is familiar with such steps and corresponding techniques, and therefore, would know how to proceed to carry out said process.

The advantages of the invention will become apparent from the following examples, which are given below as mere illustrations, and are non limitative.

The skilled person will appreciate that the present invention can incorporate any number of the preferred features described above.

All citations mentioned herein are hereby incorporated by reference in their entirety.

Other embodiments of the present invention are not presented here, which are obvious to those skilled in the art, and thus are within the scope and the spirit of the present invention. The practice of the present invention will employ, unless otherwise indicated, conventional techniques of pharmacology and pharmaceutics, which are within the skill of the art.

### Examples

### Example 1: Preparation of pharmaceutical compositions (gels)

Different pharmaceutical compositions were prepared:

| **Ingredient** | **Quantity per 100 g of gel** | | |
|---|---|---|---|
| | **57 mg 4-OHT Gel:** | **114 mg 4-OHT Gel:** | **228 mg 4-OHT Gel:** |
| | **0.057% of 4-OHT** | **0.114% of 4-OHT** | **0.228% of 4-OHT** |
| 4-Hydroxy Tamoxifen | 0.057 g | 0.114 g | 0.228 g |
| Absolute ethanol EP-USP | 66.5 g | 66.5 g | 66.5 g |
| Isopropyl myristate, EP-USP | 1 g | 1g | 1 g |
| Hydroxypropylcellulose, EP- USP | 1.5 g | 1.5 g | 1.5 g |
| Phosphate Buffer (pH 7, diluted 1:4) | q.s. 100 g | q.s. 100 g | q.s. 100 g |

| | | | |
|---|---|---|---|
| EP: European Pharmacopoeia; USP: US Pharmacopoeia | | | |

The 4-OHT containing compositions are manufactured according to a multi-step mixing process.

In general, the penetration enhancer (here, isopropyl myristate) and the alcohol (here, ethanol) are mixed; 4-OHT in the desired amount is then added to the mixture, which is then combined with hydroxypropylcellulose (Klucel HF). At the end of the process, the preparation is mixed with the aqueous vehicle (here a buffered water solution). The finished product is transferred by turbine to the container/closure system. Since the drug product is light sensitive, steps involving the drug substance are performed under inactinic light.
- Preparation of an aqueous phosphate buffer, pH 7: For a 1000 g lot of aqueous buffer at pH 7, 0.85g of KH₂PO₄ and 3.46 g of Na₂HPO₄ are weighed and diluted with 1000 g of purified water. The solution is mixed in a portable mixer for at least 10 minutes.
- The primary mixer tank is checked to assure that proper cleaning procedures have been performed.
- The alcohol is added to the mixer tank under a vacuum of 800 mbar without mixing.
- The tank is charged with isopropyl myristate. The excipient container is rinsed with alcohol.
- The inactinic lights are turned on and the room lights are turned off.
- The 4-OHT is loaded into the mixer tank and the container is rinsed with alcohol.
- The solution is mixed for 20 minutes at 2,000 rpm for the turbine and at 40 rpm for the scraper.
- The mixer tank is charged with hydroxypropylcellulose (Klucel HF) under 800 mbar vacuum, with the turbine at 2,000 rpm. The turbine is stopped at the end of the operation.
- The material is mixed for 20 minutes using the alternated mode:
   o Turbine at 2,000 rpm during 20 seconds and 600 rpm during 20 seconds.
   o Scraper : 40 rpm /min
- The turbine is stopped at the end of the operation.
- The phosphate buffer is transferred into the mixer tank under 800 mbar vacuum and with the scraper at 40 rpm.
- The product is mixed for 20 minutes using the alternated mode :
   o Turbine: 2,000 rpm during 20 seconds and 600 rpm during 20 seconds.
   o Scraper : 40 rpm / min
- The turbine is stopped at the end of the operation.
- The mixer tank is placed gradually until 100 mbar of vacuum :
   o 1st step: 250 mbar
   o 2nd step: 150 mbar
   o 3rd step: 100 mbar
- Maintain the vacuum at 100 mbar for 2 minutes with the scraper at 40 rpm.
- The product is mixed for 10 minutes with the scraper at 40 rpm. The scraper is stopped at the end of the operation.
- The gel is transferred into a stainless steel tank with a cover via extraction turbine to avoid the introduction of additional air.
- The sampling is done while the transfer is being performed.

The gels thus obtained are stable, colorless, and transparent to slightly opalescent

### Example 2: Absorption studies

### Material and methods

### 4-OHT gels

Radiolabelled 4-OHT (³H) was used in the preparation of pharmaceutical compositions as described above.

Compositions (gels) with 4-OHT concentrations of 0.057%, 0.114% and 0.228% were prepared.

### In vitro dermal absorption:

### Principle

In vitro transdermal absorption is quantitatively studied on human ventral dermatomed biopsies placed in a static diffusion cell (Franz cell), which allows contacting dermis with a survival liquid (receptor fluid) in which absorption through skin is to be dosed.

### Cell

A dermal biopsy is maintained horizontally between two parts of the cell, thus delimiting two compartments:
- one epidermal compartment is comprised of a glass cylinder, having a precisely defined area of 1.77cm², placed on the upper side of the skin;
- the other, dermal, applied to the lower face of the tegument, comprises a reservoir of fixed volume carrying a lateral collection port.

The two elements are assembled via a clamp.

The lower compartment (dermal) is filled with a receptor liquid constituted of a sodium chloride solution at 9g/L supplemented with bovine serum albumin at 15g/L.

At each time point, the survival liquid is entirely sampled out by the lateral collection port and is replaced by fresh liquid.

The lower part of the cell is thermostated at 37°C. Homogeneity of the temperature and the content in the receptor fluid, is maintained by stirring (magnetic stirrer).

The upper part (epidermal compartment) is open towards the exterior, thus exposing the epidermal surface to the air in the laboratory.

### Preparation of human ventral dermatomed skin dermal biopsies:

These are samples from human ventral skin from plastic surgery from white donors. Skin is kept at -20°C before use. Adherent sub-dermal fat is removed with a scalpel, and skin is brought to a thickness of about 0.5mm with a dermatome. For each formulation tested and each volume, 12 franz cells were set up and 3 different donors skin samples are equally distributed between the 12 cells.

### General protocol

Franz cells are usually installed the day before loading the formulation to be studied. The epidermal compartment is contacted with the atmosphere in the laboratory, the dermal compartment is thermostated to 37°C and the skin is contacted with albuminated physiological serum for about 17 hours.

The desired amount of gel (5µL, 10µL or 20µL) is applied with a micropipette onto the whole of the surface of the epidermis delimited by the glass cylinder. Sampling from the liquid contained in the dermal compartment is carried out via the lateral collection port at time point 24h.

### Radioactivity measurements

Detection is carried out by liquid scintillation using a particle counter Packard-tricarb 2900 TR.

### Preparation of radioactive samples:

The receptor liquid sampled from the lower compartment of the diffusion cells is directly incorporated in 15mL of liquid scintillation cocktail (Picofluor 40R, Packard) and metered for radioactivity measurement.

### Radioactivity measurements:

Metering rate is corrected, as far as quenching is concerned, by the method of the external calibration, in order to obtain disintegrations per minute (dpm) accounting for the real activity of each sample. The background is deducted for each sample in cpm. For each scintillation liquid, a specific quenching curve is established.

Results are expressed in weight or percentage of substance found in the samples with respect to the administered amount, determined from the metering rates of suitably diluted calibrations.

### Protocol

For a given concentration in 4-OHT in the composition and a given volume of composition applied onto the skin, average results are given, which correspond to 11-12 experimental determinations and are associated to the standard deviation (Sd).

### Results

### In vitro 4-OHT penetration in Franz cells

Average values +/- Sd of 4-OHT amounts in receptor liquid at 24h:

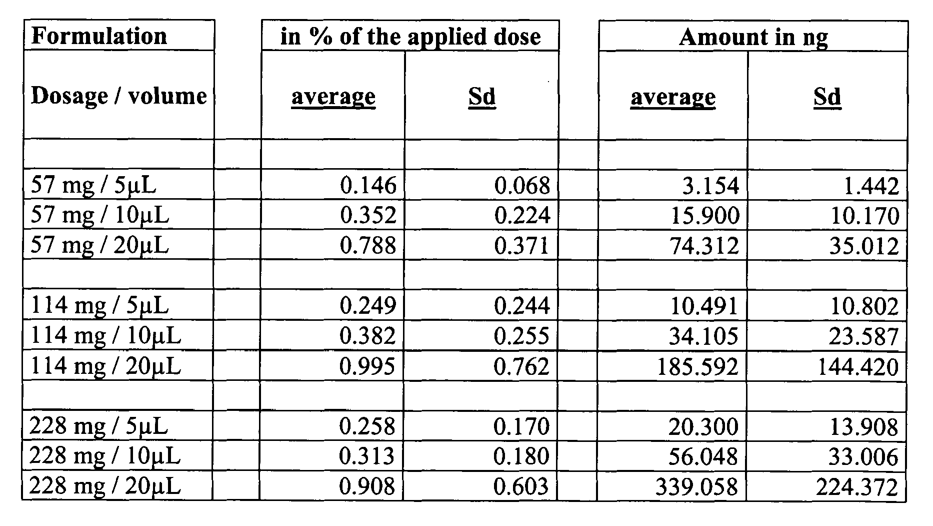

For example, in the above table, "114 mg / 10 µL" means "application on a skin surface of 1.77 cm² of 10 µL of a gel formulation, wherein said gel formulation contains 114 mg of 4-OHT per 100g of gel (0.114% of 4-OHT).

The above results show that the amount of absorbed 4-OHT is far better controlled with the 0.114% and 0.228% compositions than with the 0.057% composition for the same amount of 4-OHT applied:
For the 0.057% composition, when increasing 4-fold the application volume (from 5µL to 20µL), the percentage of absorption is unexpectedly increased about 5-fold. This results in a 23.5-fold increase in terms of absorbed amount of 4-OHT, although the applied amount of 4-OHT was only increased 4-fold (the application volume having been increased from 5 to 20 µl). In other terms, when increasing 4-fold the applied amount of 4-OHT, the actual absorbed amount is multiplied by a factor 23.5.
As a conclusion, with the 0.057% composition, there is a great variability in the amount of absorbed 4-OHT, and the absorption is not linear with the volume applied. This effect is surprisingly decreased with the 0.114% and 0.228% compositions.
For example, with the 0.228% composition, increasing 4-fold the application volume (from 5µL to 20µL) results in a more modest increase in the 4-OHT absorption percentage (about 3-fold) and the increase in the amount of absorbed 4-OHT is only 16.6-fold.

This feature is particularly advantageous where the compositions of the invention are to be applied onto the breast:
The above described experiment show that the amount of absorbed 4-OHT varies to a lesser extent as a function of volume with the 0.114% and 0.228% compositions, as compared to the 0.057% composition.
The above experiments were all conducted on the same skin surface area (1.77cm²). Hence this protocol (varying the volume of gel application for a same surface area) reflects what would happen when applying a same amount of gel onto varying skin surface areas. This is precisely the issue when the compositions are to be applied onto breast skin.

Advantageously, the compositions of the invention allow an improved compliance by the patient: for the same therapeutic dose to be applied, with the 0.228% and the 0.114% compositions, the amount of composition / gel to be applied is decreased, thus requiring a daily application, instead of bi-daily. As could be expected, it can be seen that applying the same amount of 4-OHT (either applying 10 µl of the 0.114% formulation or applying 5 µl of the 0.228% formulation), results in a similar amount of absorbed 4-OHT (34±24 versus 20±14; p>0.05, Mann Whitney). However, in view of the non linear increased absorption with a higher volume of the 0.057% formulation as discussed above, applying the same amount of 4-OHT using 20 µL of the 0.057% formulation or using 10 µL of the 0.114% formulation results in significantly different amounts of absorbed 4-OHT (74 ±35 versus 34±24; p=0.004, Mann Whitney); similarly, applying the same amount of 4-OHT using 20 µL of the 0.057% formulation or using 5 µL of the 0.228% formulation results in significantly different amounts of absorbed 4-OHT (74 ±35 versus 20±14; p=0.001, Mann Whitney). Thus, it can be seen that patients with smaller breasts, who are to apply the 0.057% formulation under a thick layer (equivalent to 20 µL per 1.77 cm² Franz cell), are overexposed as compared to patients applying the same formulation in a same amount/volume on a larger skin surface area. Patients with smaller breasts would therefore benefit from the formulations according to the present invention, as their systemic exposure, and the potential associated side effects, are advantageously reduced.

## Claims

1. Pharmaceutical composition comprising:
- 0.105 - 0.950 % of 4-hydroxy tamoxifen,
- 50 - 75% of a least one C2-C6 alcohol,
- 0.1 - 5.0 % of at least one gelling agent,
- 0.1 - 5.0 % of at least one penetration enhancer,
- 20 - 50% of an aqueous vehicle,
wherein percentages (%) are weight to weight of the composition.

2. Pharmaceutical composition according to claim 1, comprising:
- 0.205 - 0.950 % of 4-hydroxy tamoxifen,
- 50-75% of a least one C2-C6 alcohol,
- 0.1 - 5.0 % of at least one gelling agent,
- 0.1 - 5.0 % of at least one penetration enhancer,
- 20 - 50 % of an aqueous vehicle,
wherein percentages (%) are weight to weight of the composition.

3. Pharmaceutical composition according to any one of claims 1-2, comprising:
- 0.220 - 0.350 %, preferably about 0.228 % of 4-hydroxy tamoxifen,
- 60 - 75 % of ethanol,
- 0.5 - 1.5 % of hydroxypropylcellulose,
- 0.4 - 2.0 % of myristate isopropyl,
- 20 - 40 % of phosphate buffer,
wherein percentages (%) are weight to weight of the composition.

4. Pharmaceutical composition according to any one of claims 1-3, wherein said C2-C6 alcohol is selected from the group consisting of ethanol, propan-1-ol and propan-2-ol, and mixtures thereof.

5. Pharmaceutical composition according to any one of claims 1-4, wherein said gelling agent is selected from the group consisting of acrylic acid-based polymers, cellulosics, and mixtures thereof.

6. Pharmaceutical composition according to any one of claims 1-5, wherein said gelling agent comprises at least one gelling agent selected from the group consisting of carboxymethylcelluloses, hydroxypropylcelluloses, hydroxyethylcelluloses, ethylcelluloses, hydroxymethylcelluloses, hydroxypropylmethylcelluloses, and mixtures thereof.

7. Pharmaceutical composition according to any one of claims 1-6, comprising isopropyl myristate.

8. Pharmaceutical composition according to any one of claims 1-7, wherein said aqueous vehicle comprises at least one base, preferably selected from the group consisting of triethanolamine, sodium hydroxide, ammonium hydroxide, potassium hydroxide, arginine, aminomethylpropanol, tromethamine, and mixtures thereof.

9. Pharmaceutical composition according to any one of claims 1-8, wherein said aqueous vehicle comprises at least one buffer, preferably selected from the group consisting of citrate, tris maleate, phosphate buffers, and mixtures thereof.

10. Pharmaceutical composition according to any one of claims 1-9, having a pH of 7.5-10.0.

11. Gel useful for transdermal or transcutaneous delivery comprising a pharmaceutical composition according to any one of claims 1-10.

12. Dose packet, unit dose packet or multiple dose packet containing a pharmaceutical composition according to any one of claims 1-10 or a gel according to claim 11.

13. Dispenser, e.g. with hand pump, containing a pharmaceutical composition according to any one of claims 1-10 or a gel according to claim 11.

14. Process for preparing a pharmaceutical composition according to any one of claims 1-10 or a gel according to claim 11, comprising the steps of:
- preparing a mixture containing at least one C2-C6 alcohol, and at least one penetration enhancer;
- adding 4-hydroxy tamoxifen in the desired amount, and mixing;
- adding at least one gelling agent, and mixing again;
- adding at least one aqueous vehicle, and mixing again.

15. Pharmaceutical composition according to any one of claims 1-10 or gel according to claim 11, for use in treating and/or preventing benign breast diseases, scars and keloids, gynecomastia, breast cancer, mastalgia, and conditions involving dense breast tissue.
